(19) Europäisches Patentamt
European Patent Office
Office européen des brevets

(11) Veröffentlichungsnummer: **0 186 075**
**B1**

(12) # EUROPÄISCHE PATENTSCHRIFT

(45) Veröffentlichungstag der Patentschrift: **28.02.90**

(51) Int. Cl. [5]: **C 07 C 47/445,** C 07 C 45/50

(21) Anmeldenummer: **85115956.6**

(22) Anmeldetag: **13.12.85**

(54) **Verfahren zur Herstellung von 8- und 9-Formyl-tricyclo-(5,2,1,0, 2,6)-decen-3.**

(30) Priorität: **22.12.84 DE 3447030**

(43) Veröffentlichungstag der Anmeldung:
**02.07.86 Patentblatt 86/27**

(45) Bekanntmachung des Hinweises auf die Patenterteilung:
**28.02.90 Patentblatt 90/09**

(84) Bennante Vertragsstaaten:
**AT BE CH DE FR GB IT LI LU NL SE**

(56) Entgegenhaltungen:
**DE-A-2 627 354**
**DE-A-2 654 799**
**FR-A-2 489 308**
**GB-A-2 054 578**

(73) Patentinhaber: **HOECHST AKTIENGESELLSCHAFT**
**Postfach 80 03 20**
**D-6230 Frankfurt am Main 80 (DE)**

(72) Erfinder: **Cornils, Boy, Dr. Dipl.-Chem.**
**Friedrich-Ebert-Strasse 45**
**D-4220 Dinslaken (DE)**
Erfinder: **Konkol, Werner, Dr. Dipl.-Chem.**
**Lützowstrasse 40a**
**D-4200 Oberhausen 11 (DE)**
Erfinder: **Bach, Hanswilhelm, Dr. Dipl.-Chem.**
**Alleestrasse 56**
**D-4100 Duisburg 11 (DE)**
Erfinder: **Gick, Wilhelm, Dr. Dipl.-Chem.**
**Im Buschhuck 8**
**D-4100 Duisburg 74 (DE)**
Erfinder: **Wiebus, Ernst**
**Ferdinandstrasse 77**
**D-4200 Oberhausen 11 (DE)**
Erfinder: **Bahrmann, Helmut, Dr. Dipl.-Chem.**
**Rohstrasse 48**
**D-4236 Hamminkeln-Brünen (DE)**

**EP 0 186 075 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft ein Verfahren zur Herstellung von 8- und 9-Formyl-tricyclo (5, 2, 1, $0^{2,6}$)-decen-3 durch Hydroformylierung von Dicyclopentadien in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen, darüber hinaus werden bei Einsatz acyclischer endständiger Olefine vorzugsweise n-Aldehyde und nur in untergeordnetem Maße iso-Aldehyde gebildet. Schließlich ist auch die Hydrierung der Olefine zu gesättigten Kohlenwaserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form modifizierter Hydridorhodiumcarbonyle eingesetzt, die zusätzliche Liganden enthalten.

Diese Liganden können im Katalysatorsystem auch im Überschuß vorhanden sein. Als Liganden haben sich besonders tertiäre Phosphine oder Phosphite bewährt.

Der Einsatz solcher Katalysatoren für die Hydroformylierung von Dicyclopentadien unter Bildung eines ungesättigten Monoaldehyds ist in der DE-2 654 799 C2 beschrieben.

Probleme bei Verwendung der genannten Katalysatoren bereitet jedoch die Abtrennung der Reaktionsprodukte und die Wiederge-winnung der im Reaktionsprodukt homogen gelösten Katalysatoren. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydrodormylierung niedriger Olefine, d. h. Olefine mit bis zu etwa 5 Kohlenstoffatomen im Molekül beschritten werden. Außerdem hat sich gezeigt, daß die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z. B. in der DE-PS-2 627 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der i Hydroformylierungsreaktion erfolgt bei dieser Verfahrensvariante einfach durch Trennung von wässriger und organischer Phase, d. h. ohne Destillation und damit ohne zusätzliche thermische Verfahrensschritte. Ein Nachteil dieses Verfahrens ist, daß bei Einsatz höherer Olefine d. h. solcher mit mehr als etwa vier Kohlenstoffatomen, also auch bei Einsatz von Dicyclopentadien, der Umsatz merklich zurückgeht.

Dieser Rückgang wird durch die Abnahme der Löslichkeit der höheren Olefine in der wässrigen Katalysatorlösung verursacht, denn die Reaktion zwischen den Reaktanten läuft in der wässrigen Phase ab.

Es bestand daher die Aufgabe, ein Verfahren zu entwickeln, das einen annähernd vollständigen Umsatz von Dicyclopentadien mit Kohlenmonoxid und Wasserstoff bei hoher Selektivität zu 8- und 9-Formyltricyclo (5, 2, 1, $0^{2,6}$)-decen-3 sicherstellt und eine einfache und leichte Abtrennung und Wiedergewinnung des Katalysatorsystems ermöglicht.

Erfindungsgemäß wird die vorstehend beschriebene Aufgabe gelöst durch ein Verfahren zur Herstellung von 8- und 9-Formyltricyclo (5, 2, 1, $0^{2,6}$)-decen-3 durch Umsetzung von Dicyclopentadien mit Kohlenmonoxid und Wasserstoff in flüssiger Phase in Gegenwart von Wasser sowie Rhodium in metallischer Form oder als Verbindung und eines wasserlöslichen Arylphosphins. Es ist dadurch gekennzeichnet, daß die Umsetzung bei Temperaturen von 80 bis 140 °C und Drücken von 1 bis 20 MPa durchgeführt wird und das wasserlösliche Phosphin der allgemeinen Formel

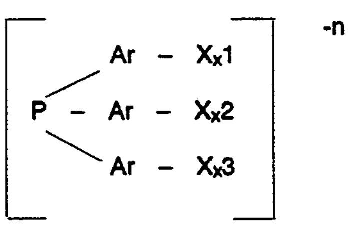 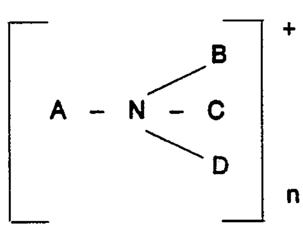

folgt, wobei Ar für eine Arylgruppe und X für eine Sulfonsäuregruppe steht, $x^1$, $x^2$, $x^3$ 0 oder 1 bedeutet mit der Maßgabe, daß mindestens eine Zahl $x^1$, $x^2$ oder $x^3$ 1 ist, A für einen Alkyl- oder Aralkylrest mit 7 bis l8 Kohlenstoffatomen steht und B, C, D geradkettige oder verzweigte Alkylreste mit 1 bis 4 Kohlenstoffatomen sind, und n eine ganze Zahl von 1 bis 3 ist.

Überraschenderweise hat sich gezeigt, daß bei Einhaltung der ausgewählten Reaktionsbedingungen und Einsatz von wasserlöslichen Phosphinen entsprechend dem erfindungsgemäßen Verfahren Dicyclopentadien weitgehend und mit hoher Selektivität zu einem Gemisch der ungesättigten Monoaldehyde umgesetzt wird.

Gleichzeitig wird aber auch die mit dem organischen Reaktionsprodukt ausgetragene Rhodium- und Phosphinmenge erheblich reduziert.

Dieses Verhalten des im erfindungsgemäßen Verfahren eingesetzten Katalysatorsystems ist überraschend. Aufgrund der hohen Aktivität des Katalysators war nicht vorauszusehen, daß die Umsetzung des Diolefins auf der Stufe der Monoformylverbindung stehen bleibt und nicht zur Diformylverbindung führt. Überdies ist die ausgezeichnete Selektivität der Reaktion bemerkenswert, da der Formylrest ausschließlich in die 8- und 9-Stellung eintritt, während Isomere, in denen die 3- oder 4-Stellung der tricyclischen Verbindung durch die Formylgruppe besetzt ist, nicht auftreten. Im Vergleich zum Verfahren, das der DE-2 654 799 C2 zugrunde liegt, zeichnet sich die neue Arbeitsweise dadurch aus, daß bereits unter milden Reaktionsbedingungen und mit geringen Katalysatorkonzentrationen hohe Umsätze erzielt werden. Zudem kann der Katalysator wiederholt rezirkuliert und kontinuierlich mit frischem Dicyclopentadien umgesetzt werden. Auch bei mehr als sechzigfachem Einsatz konnte eine Abnahme seiner Aktivität nicht beobachtet werden. Der Katalysatorverbrauch bezogen auf eingesetztes Olefin ist daher äußerst gering.

Unter den wasserlöslichen Phosphinen der oben wiedergegebenen allgemeinen Formel werden im Rahmen der neuen Arbeitsweise insbesondere Verbindungen emgesetzt, in denen Ar eine Phenyloder Naphthylgruppe, die Summe von $x^1$, $x^2$ und $x^3$ 2 oder 3 und B, C und D die gleichen geradkettigen oder verzweigten Alkylgruppen mit 1 bis 4 Kohlenstoffatomen bedeuten.

Beispiele für wasserlösliche Phosphine, die sich zur Durchführung des neuen Verfahrens eignen, sind Triphenyldi- und trisulfonate, die als Kationen Trimethylcetylammonium, Trimethyldodecylammonium, Tributyldodecylammonium, Dodecylethyldimethylammonium, Triethylbenzylammonium, Trimethylbenzylammonium enthalten.

Die Herstellung der in dem beanspruchten Verfahren verwendeten Phosphine ist bekannt. Ausgangsverbindungen sind sulfonierte Triarylphosphine, die durch Behandlung von Triarylphosphinen mit Oleum erhalten werden.

Zweckmäßig gewinnt man zunächst aus dem mit Wasser verdünnten Sulfonierungsgemisch in Wasser unlösliche, in organischen Lösungsmitteln lösliche Aminsalze. Diese werden durch Behandlung mit einem quartären Ammoniumhydroxid in das gewünschte Onium-Salz des sulfonierten Triarylphosphins überführt.

Nach der in der EP-0 104 967 A1 beschriebenen Arbeitsweise gewinnt man das Onium-Salz des sulfonierten Triarylphosphins aus dem Sulfonierungsprodukt durch Extraktion mit Estern verschiedener Säufen des Phosphors oder mit Phosphinoxiden oder Diphosphindioxiden. Anschließend behandelt man die organische Phase mit wässriger TetraalkylammoniumhydroxidLösung und erhält so das Onium-Salz.

Das beanspruchte Hydroformylierungsverfahren kann absatzweise ausgeübt werden. Besondere Vorteile bietet seine kontinuierliche Durchführung. Hierbei durchläuft der den Reaktor verlassende Produktstrom ohne Kühlung einen Phasentrenner oder eine Zentrifuge, wobei die obere organische Produktphase von der unteren Katalysatorphase abgetrennt wird. Die untere Phase wird dann, gegebenenfalls nach Durchlaufen eines Wärmetauschers, zur Abführung eines Teils der Reaktionswärme zusammen mit frischem Dicyclopentadien und Synthesegas in den Reaktor zurückgeführt. Die mit dem organischen Produkt ausgetragene Rhodiummenge ist gering.

Die Reaktion läuft unter verhältnismäßig milden Bedingungen bei Temperaturen von 80 bis 140°C, vorzugsweise 100 bis 130 °C und Drücken von 1 bis 20 MPa, insbesondere 2 bis 5 MPa. Die Reaktionsdauer beträgt 1 - 5 h, entsprechend einer durchschnittlichen Raumgeschwindigkeit von 1 - 0,2 V/V.h

Als Ausgangsstoff kann sowohl reines als auch technisches Dicyclopentadien eingesetzt werden. So hat sich das erfindungsgemäße Verfahren selbst bei Verwendung von technischem Dicyclopentadien mit einer Reinheit von 94 % sehr gut bewährt.

Der Katalysator kann dem Reaktionssystem präformiert zugesetzt werden. Mit gleich gutem Erfolg läßt er sich aber auch aus den Komponenten Rhodium oder Rhodiumverbindung und der wässrigen Lösung des quartären Ammoniumsalzes des sulfonierten Triarylphosphins unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethyl-hexanoat oder unlösliche Verbindungen wie Rhodiumoxid $Rh_2O_3$ eingesetzt werden.

Die Rhodiumkonzentration in der Katalysatorlösung kann sich in weiten Bereichen bewegen und zwischen 10 und 5000, vorzugsweise 100 bis 500 Gew.-ppm betragen. Eine Erhöhung der Rhodiumkonzentration führt zu einer Steigerung der Raum-ZeitAusbeute. Oberhalb etwa 1000 Gew.-ppm Rhodium ist der Anstieg der Raum-Zeit-Ausbeute mit zunehmender Rhodiumkonzentration jedoch geringer als in niedrigeren Konzentrationsbereichen. Das bedeutet, daß das eingesetzte Rhodium zunehmend weniger ausgenutzt wird.

Darüber hinaus hat sich gezeigt, daß die chemische Beständigkeit der als Katalysator wirkenden Rhodiumcarbonylphosphin-Komplexverbindungen bei hoher Rhodiumkonzentration abnimmt, so daß die Lebensdauer des Katalysators im kontinuierlichen Einsatz beeinträchtigt wird.

Es hat sich bewährt, die in Wasser gelösten Rhodiumcarbonylphoshin-Komplexverbindungen durch überschüssiges, nicht komplex gebundenes Phosphin zu stabilisieren. Der Anteil an freiem Phosphin, bezogen auf 1 g-Atom Rhodium, kann zwischen etwa 10 bis etwa 1000 mol betragen, das Verhältnis ist stark abhängig von den Reaktionsbedingungen z. B. von der Rhodiumkonzentration. Niedrige Rhodiumkonzentrationen erfordern in der Regel einen relativ höheren Phosphinüberschuß als höhere Rhodiumkonzentrationen. Bei Konzentrationen von 100 - 400 Gew.-ppm Rhodium hat es sich bewährt, je g-Atom Rhodium 50 bis 800 mol Phosphin einzusetzen.

Der Zusatz von nicht komplexgebundenem freiem Phosphin hat neben der Stabilisierung der Rhodiumcarbonylphosphin-Komplexe auch eine Erhöhung der Salzkonzentration in der wässrigen Katalysatorlösung zur Folge. Dadurch wird die Löslichkeit der Reaktanten im Reaktionsmedium und damit auch der Umsatz beeinflußt. Es empfiehlt sich daher, die Konzentration des wasserlöslichen Phosphins auf Werte zwischen 10 und 90, insbesondere zwischen 30 und 60 Gew.-% (bezogen auf die wässrige Lösung) einzustellen.

Der pH-Wert der wässrigen Katalysatorlösung soll nicht geringer als 2 sein. Bewährt haben sich pH-Werte von 2 bis 13, vorzugsweise 4 bis 10.

Die Zusammensetzung des, d. h. das Verhältnis von Kohlenmonoxid zu Wasserstoff kann in weiten Grenzen variiert werden. Im allgemeinen setzt man Synthesegas ein, in dem das Volumenverhältnis von Kohlenmonoxid zu Wasserstoff 1 : 1 beträgt oder von diesem Wert nur wenig abweicht.

Die im erfindungsgemäßen Verfahren eingesetzten Oniumphosphinsalze steigern nicht nur den Dicyclopentadien-Umsatz, sondern erhöhen auch die Löslichkeit von Wasser in dem aus 8- und 9-Formyl-tricyclo (5, 2, 1, 0 $^{2,\ 6}$)-decen-3 bestehenden Reaktionsgemisch bei höheren Temperaturen beträchtlich. Daher scheiden sich aus dem Reaktionsgemisch nach Abkühlung 1 - 10 Gew.-% und insbesondere 3 - 7 Gew.-% Wasser ab, das rezirkuliert wird. Außerdem empfiehlt es sich, die noch in dem ungesättigten Aldehyd physikalisch gelöste und mit diesem ausgetragene Wassermenge kontinuierlich bzw. absatzweise zu ergänzen. Um eine Oxidation des Katalysators zu verhindern, hat sich hierzu die Verwendung von luftfreiem bzw. sauerstofffreiem Kondensat bewährt. In einer besonderen Ausführungsform kann auch Waschwasser aus der Wäsche des Oxorohproduktes zur Abtrennung restlicher Phosphor- und Rhodiumspuren vor der destillativen Weiterverarbeitung verwendet werden.

Die Alkohol-, Kohlenwasserstoff- und Dialdehydbildung ist minimal.

Die folgenden Beispiele erläutern die Erfindung näher.

**Beispiel 1 (Vergleich)**

In einem 1 1 Autoklaven mit Tauchstutzen werden 470 g einer wässrigen Lösung, die 16,4 Gew.-% Tri(m-sulfophenyl)phosphin-Na-Salz sowie 2,4 Gew.-% Di(m-sulfophenyl)phenylphos-phin-Na-Salz und 400 ppm Rh als Rh-Acetat enthält, vorgelegt. Darauf wird Synthesegas ($CO/H_2$ = 1 : 1) bis zu einem Druck von 2,5 MPa aufgepreßt. Die Reaktionslösung wird bei 125°C 3 h unter Rühren mit dem Synthesegas behandelt. Man kühlt auf etwa 30°C ab, beendet das Rühren und drückt nach einer Absetzzeit von 15 min die überschüssige Lösung, 151 g, über den Tauchstutzen heraus. Sie wird analysiert.

Zu der im Autoklaven verbleibenden Lösung werden unter Rühren über eine Druckpumpe 200 g technisches Dicyclopentadien, das zusammen 94,3 Gew.-% exo- und endo-Dicyclopentadien enthält, gepumpt. Unter Aufrechterhaltung eines Druckes von 2,5 MPa wird 3 h auf 125°C erhitzt. Danach läßt man auf 30°C abkühlen und absitzen. Die überstehende organische Phase wird über den Tauchstutzen herausgedrückt, darauf gewogen und gaschromatographisch untersucht. Die Hydroformylierung wird insgesamt viermal wiederholt, wobei im wesentlichen die gleichen Ergebnisse resultieren.

Nach Beendigung des Versuches werden 293 g Katalysatorlösung zurückerhalten. Die restliche Lösung wird zusammen mit dem Oxorohprodukt ausgetragen. Die Versuchsergebnisse sind in Tabelle 1 zusammengestellt. Beispiel 1 zeigt, daß bei Verwendung des Na-Salzes des Tri-(m-sulfophenyl)phosphins nur ein ungenügender Teilumsatz erzielt wird.

**Tabelle 1**

| Anzahl der Hydroformylierungen | 1 | 2 | 3 | 4 | 5 |
|---|---|---|---|---|---|
| Umsatz (Gew. -% nach GC) | 41 | 45 | 31 | 31 | 34 |
| Gehalt an Tricyclodecenal (Gew.-% nach GC) | 25,41 | 37,23 | 24,16 | 24,69 | 26,53 |
| Gehalt an Tricyclodecendial (Gew.-% nach GC) | 0,06 | 0,18 | 0,05 | 0,04 | 0,03 |
| Menge an organischer Phase (g) | 214 | 213 | 212 | 240 | 214 |

**Beispiel 2**

Beispiel 1 wird wiederholt mit dem Unterschied, daß anstelle des Na-Salzes 370 g einer wäßrigen Lösung des Trimethylbenzylammonium-salzes von Tri-(m-sulfophenyl)phosphin mit einem P(III)-Gehalt von 0,320 Gew.-% und 330 Gew.-ppm Rh als Rh-Acetat in die Hydroformylierung eingesetzt werden. Nach der Synthesegasbehandlung werden 86 g Katalysatorlösung für analytische Zwecke über den Tauchstutzen herausgenommen. Die Hydroformylierungen werden nach dem 6. Einzelversuch unterbrochen, ohne das eine Abnahme des Umsatzes bzw. der Selektivität bezüglich der Bildung von ungesättigten Monoaldehyd eintritt. Die Versuchsergebnisse sind in Tabelle 2 zusammengestellt.

**Tabelle 2**

| Anzahl der Hydroformylierungen | 1 | 2 | 3 | 4 | 5 | 6 |
|---|---|---|---|---|---|---|
| Umsatz (Gew. -% nach GC) | 50,8 | 98,5 | 97.2 | 97,8 | 96,6 | 98,3 |
| Gehalt an Tricyclodecenal | 46,6 | 91,9 | 92,3 | 93,2 | 90,2 | 92,6 |

| | | | | | | |
|---|---|---|---|---|---|---|
| (Gew.-% nach GC) | | | | | | |
| Gehalt an Tricyclodecandial (Gew.-% nach GC) | 2,9 | 2,8 | 2,9 | 3,6 | 2,7 | 3,4 |
| Menge an organischer Phase (g) | 256 | 187 | 255 | 234 | 245 | 253 |
| Menge an wäßriger Katalysasatorphase (g) | 25 | 2 | 1 | - | - | - |

Zur Ermittlung der mit der organischen Phase insgesamt ausgetragenen Rhodium- und Phosphormengen sowie zur Bestimmung der Ausbeute wird ein Teil (808 g) der dem Reaktor in den einzelnen Versuchen entnommenen organischen Anteile vereinigt, zur Entfernung restlicher Katalysatormengen mit entionisiertem und luftfreiem Wasser gewaschen und destilliert. Bei 137 - 142°C Sumpftemperatur und 116 - 118°C Kopftemperatur werden bei 1,3 kPa und einem Rücklaufverhältnis von 10 : 1 30,2 g Vorlauf, 633,1 g Hauptlauf, der aus ungesättigtem Monoaldehyd in 98,5-%-iger Reinheit besteht, sowie 70,1 g Destillationsrückstand erhalten. Die Destillationsverluste betragen 4,2 %.

Der Destillationsrückstand enthält 0,08 Gew.-ppm Rh und 0,05 Gew.-ppm Phosphor (jeweils bezogen auf die ursprüngliche organische Phase).

Ein Vergleich der Beispiele 1 und 2 zeigt, daß unter Anwendung der erfindungsgemäßen Oniumphosphinsalze Tricyclodecenal in hoher Ausbeute hergestellt werden kann. Beispiel 2 verdeutlicht darüber hinaus, daß sich die Rhodiumund Phosphorverluste, bezogen auf umgesetztes Dicyclopentadien, in einem technisch und wirtschaftlich vertretbaren Rahmen bewegen.

## Patentansprüche

1. Verfahren zur Herstellung von 8- und 9-Formyltricyclo-(5, 2, 1, $0^{2,6}$)-decen-3 durch Umsetzung von Dicyclopentadien mit Kohlenmonoxid und Wasserstoff in flüssiger Phase in Gegenwart von Wasser sowie Rhodium in metallischer Form oder als Verbindung und eines wasserlöslichen Arylphosphins, dadurch gekennzeichnet, daß die Umsetzung bei Temperaturen von 80 bis 140°C und Drücken von 1 bis 20 MPa durchgeführt wird und das wasserlösliche Phosphin der allgemeinen Formel

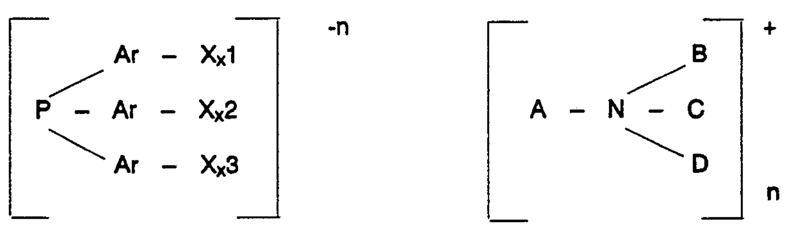

folgt, wobei Ar für eine Arylgruppe und X für eine Sulfonsäuregruppe steht, $x^1$, $x^2$, $x^3$ 0 oder 1 bedeutet mit der Maßgabe, daß mindestens eine Zahl $x^1$, $x^2$ oder $x^3$ ist, A für einen Alkyl- oder Aralkylrest mit 7 bis 18 Kohlenstoffatomen steht und B, C, D geradkettige oder verzweigte Alkylreste mit 1 bis 4 Kohlenstoffatomen sind, und n eine ganze Zahl von 1 bis 3 ist.

2. Verfahren nach Anspruch 1 dadurch gekennzeichnet, daß als wasserlösliches Phosphin Verbindungen eingesetzt werden, in denen Ar eine Phenyl- oder Naphthylgruppe, die Summe von $x^1$, $x^2$, $x^3$ 2 oder 3 und B, C und D die gleichen geradkettigen oder verzweigten Alkylgruppen mit 1 bis 4 Kohlenstoffatomen bedeuten.

## Claims

1. A process for preparing 8- and 9-formyltricyclo-(5, 2, 1, $0^{2,6}$)-decene-3 by the reaction of dicyclopentadiene with carbon monoxide and hydrogen in the liquid phase in the presence of water, rhodium in metallic form or as a compound and a watersoluble arylphosphine, characterised in that the reaction takes place at temperatures of 80 to 140°C and pressures of 1 to 20 MPa and the water-soluble phosphine has the general formula:

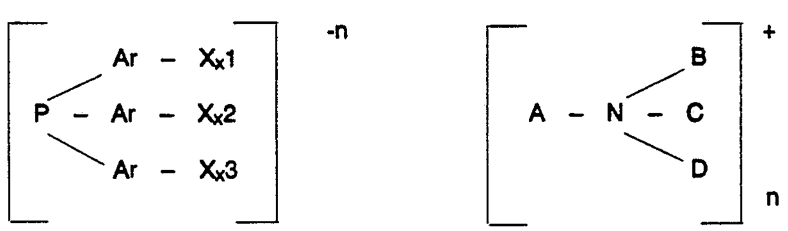

where Ar stands for an aryl group and X for a sulfonic acid group, $x^1$, $x^2$ and $x^3$ denote 0 or 1 subject to the condition that at least one number $x^1$, $x^2$ or $x^3$ is 1, A stands for an alkyl or aralkyl radical having 7 to 18 carbon atoms and B, C, D are straight-chain or branched alkyl radicals having 1 to 4 carbon atoms, and n is an integer from 1 to 3.

2. A process according to claim 1, characterised in that compounds are used as the water-soluble phosphine, where Ar stands for a phenyl or naphthyl group, the sum of $x^1$, $x^2$ and $x^3$ is 2 or 3 and B, C and D denote the same straight-chain or branched alkyl groups having 1 to 4 carbon atoms.

## Revendications

1. Procédé pour la fabrication de 8- et 9-formyltricyclo-[5. 2. 1. $0^{2,6}$]-décènes-3 par réaction du dicyclopentadiène avec le monoxyde de carbone et l'hydrogène en phase liquide en présence d'eau et et rhodium sous forme métallique ou sous forme de composé et d'une arylphosphine soluble dans l'eau, caractérisé en ce que la réaction est mise en oeuvre à des températures de 80 à 140°C et sous des pressions de 1 à 20 MPa et la phosphine soluble dans l'eau répond à la formule générale

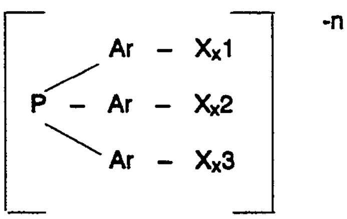

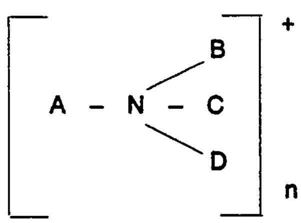

dans laquelle Ar représente un groupe aryle et X un groupe acide sulfonique, $x^1$, $x^2$ et $x^3$ représentent 0 ou 1 avec la condition que l'un au moins des nombres $x^1$, $x^2$ et $x^3$ est égal à 1, A représente un reste alkyle ou arylalkyle en $C_7$-$C_8$ et B, C et D représentent des restes alkyles à chaîne droite ou ramifiée en $C_1$-$C_4$ et n est un nombre entier compris entre 1 et 3.

2. Procédé selon la revendication 1, caractérisé en ce que l'on utilise comme phosphines solubles dans l'eau des composés dans lesquels Ar représente un groupe phényle ou naphtyle, la somme de $x^1$, $x^2$ et $x^3$ est égale à 2 ou 3 et B, C et D représentent les mêmes groupes alkyles à chaîne droite ou ramifiée en $C_1$-$C_4$.